# EUROPEAN PATENT SPECIFICATION

(11) **EP 2 525 785 B1**
(45) Date of publication and mention of the grant of the patent: **25.03.2015**
(21) Application number: 10798647.3
(22) Date of filing: 01.11.2010
(51) Int. Cl.: A61K 9/20, A61K 9/28, A61K 31/407

(54) **SUSTAINED RELEASE TABLET FORMULATION CONTAINING ETODOLAC**
TABLETTE MIT VERZOGERTER WIRKSTOFFABGABE ENTHALTEND ETODOLAC
COMPRIME A LIBERATION PROLONGEE CONTENANT DU ETODOLAC

(30) Priority: 18.01.2010 TR 201008308
(43) Date of publication of application: 28.11.2012
(73) Proprietor: Turgut Laclari A.S., 34381 Istanbul (TR)
(72) Inventor: PILLI, Gulhan, 34381 Istanbul (TR); YIGIT, Arif, 34381 Istanbul (TR); DENIZ, Hilmi, 34381 Istanbul (TR); FIRAT, Omer Faruk, 34381 Istanbul (TR)
(74) Representative: Berkkam, Ayfer
(86) International application number: PCT/TR2010/000216
(87) International publication number: WO 2011/087464

(56) References cited:
- WO-A1-00/09091
- WO-A2-01/19349
- US-A- 4 657 757
- US-A- 4 966 768
- BELLAMY N: "ETODOLAC IN THE MANAGEMENT OF PAIN: A CLINICAL REVIEW OF A MULTIPURPOSE ANALGESIC", INFLAMMOPHARMACOLOGY, KLUWER ACADEMIC PUBLISHERS, DORDRECHT, NL, vol. 5, no. 2, 1 January 1997 (1997-01-01) , pages 139-152, XP008019884, ISSN: 0925-4692

## Description

### Field of the Invention

This invention relates to a method for preparing a formulation in sustained release form that comprises 2-(1,8-diethyl-4,9-dihydro-3*H*-pyrano[3,4-b]indole-1-il)acetic acid (Etodolac), which is an effective non-steroidal anti-inflammatory drug in the treatment of osteoarthritis, rheumatoid arthritis and ankylosing spondylitis symptoms and findings, in the treatment of acute gouty arthritis, acute muscular skeletal system pain, post-operative pain and dysmenorrhea by inhibiting the biosynthesis of prostaglandin.

### Prior Art

Non-steroidal anti-inflammatory drugs (NSAIDs) are preferred and used in most of the painful diseases, since they do not cause dependency and do not create a state of narcosis expressed as lethargy and confusion. The said drugs are especially beneficial in inflammation-related cases and where long time administration of analgesic drugs is needed, such as arthritis, osteoarthritis and similar rheumatismal diseases. The fact that they do not cause dependency, that they have anti-inflammatory effect and that no tolerance is - developed against their therapeutic effects increase the therapeutic value of this drug group. Most NSAIDs have antipyretic effect in addition to their analgesic effect.

In order to be able to keep the plasma dosage form of the drug at the effective dose in conventional dosage forms, frequent administration of the drug is needed. For instance, some time after the conventional dosage form is taken, the plasma concentration of the active ingredient begins to rise and after a short period of 3 or 4 hours it begins to fall and active ingredient - plasma level is kept at the effective field by taking a new dose and the treatment can be provided by repeating this process many times. However, during all these treatment the plasma level of the drug might reach to toxic field or retreat to ineffective field.

In systems where non-steroidal anti-inflammatory drugs are used, the drug dissolves slowly within a certain time range with SR (Sustained Release) forms. After a certain dose intake, the plasma level keeps constant for a desired period, so the patient does not require frequent drug intakes and successful treatment can be attained with a very low dose, since the active ingredient level of the plasma does not change.

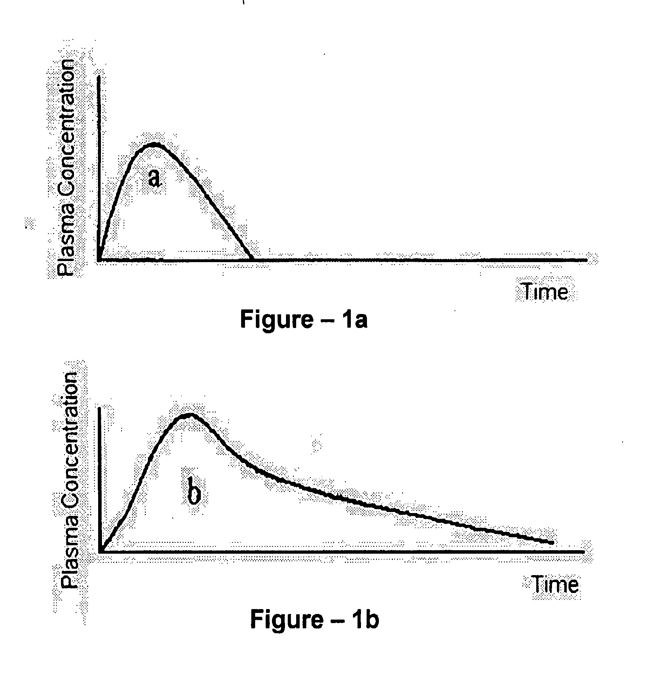

The graph demonstrating the plasma concentrations of Immediate Release (IR) drug systems and Sustained Release (SR) drug systems against time are shown in Figure 1a and Figure 1b.

In the following table taken from the article named "Pharmacokinetics of sustained-release etodolac" (Rheumatol Int. 13: S3-S5,1993) by Prof. L.Z. Benet, one of the leading research scientists in the field of Pharmacokinetics, the pharmacokinetics of multiple doses of Etodolac 600 mg Sustained Release tablet and Etodolac 300 mg Immediate Release tablets given twice a day (600 mg daily) are compared in a cross-over study where 19 healthy volunteers attended.

**Table 1: Etodolac SR and Etodolac IR Comparison**

| **Etodolac SR** | **Parameter** | **Etodolac IR** |
|---|---|---|
| 146 ± 61 | AUC₀₋₂₄(µg . h/ml) | 162 ± 51 |
| 11.9 ± 4.1 | Cₘₐₓ (µg/ml) | 20.8 ± 6.7 |
| 7.8 ± 3.2 | tₘₐₓ (h) | 1.7 ± 1.3 |
| 7.4 ± 4.4 | Cₘₐₓ/Cₘᵢₙ | 7.5 ± 3.8 |

As seen from the above table, when given in repeated doses, the Cₘₐₓ value of the immediate release tablet (20.8 ± 6.7 µg/ml) is well above the Cₘₐₓ value (11.9 ± 4.1 µg/ml) of the sustained release tablet. Cₘₐₓ value of the immediate release tablet is almost close to the double of the Cₘₐₓ value of the sustained release tablet. Although maximum plasma concentration of the sustained release tablet is lower than the immediate release tablet, time to this concentration (tₘₐₓ) is longer, which causes the active concentration to last longer. However in the immediate release tablet, time to Cₘₐₓ (tₘₐₓ) is shorter, which leads to a shorter effective time, as well.

Etodolac, which is an indole derivative, is an example of non-steroidal anti-inflammatory drugs (NSAID) and it is a drug which is especially beneficial in inflammation-related cases and where long time administration of analgesic drugs is needed, such as arthritis, osteoarthritis and similar rheumatismal diseases. The chemical formula of etodolac whose IUPAC name is "*2-(1,8-diethyl-4,9-dihydro-3H-pyrano[3,4-b]indole-1-il)acetic acid'* is given below:

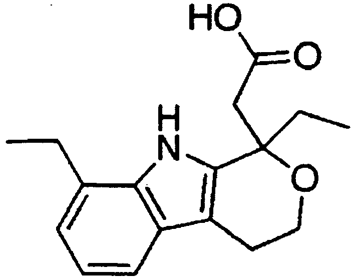

The dissolution problem of Etodolac in acidic medium has been addressed by Michelucci *et al.* and a release rate modifying agent has been added to the tablet for maintaining an alkaline environment.

There are many patent applications in the prior art of Etodolac which demonstrates anti-inflammatory, analgesic and antipyretic properties.

In the prior art, the document WO 01/19349 relates to a sustained release formulation of etodolac for once daily administration. In the said document, it is described that low solubility of Etodolac is observed below pH 3, and the solubility gradually increases with increasing pH up to 5 and then linearly increases with increasing pH up to 7.

The formulation in the document WO 01/19349 contains hydroxypropylcellulose whose sustained release formula has varying degrees of viscosity (HPC-L, HPC-M). HPC-L is an easily swellable material and it controls the initial release of the drug from the dosage form. HPC-M controls the drug release over an extended time period. Lactose is used as diluent and polyvinylpyrrolidon is used as binder.

The document US 4 966 768 in the prior art relates to a sustained release dosage form of etodolac for once daily administration. In the said document, it is intended to minimize the pH solubility dependency throughout the gastrointestinal tract with the addition of the release rate modifying agent. An admixture of a hydrophilic polymer, hydroxypropylmethylcellulose and a hydrophobic polymer, ethylcellulose is used for sustained release, and sodium dibasic phosphate is used as release rate modifying agent.

The document US 4 704 285 in the prior art relates to the use of fine particle sized hydroxypropylcellulose ether composition for delaying the release of the active ingredient.

The document WO 9912524 in the prior art relates to modified release multiple-units. The said document provides a first fraction of quick release multiple-units, and a second fraction of coated modified release multiple-units.

The document TR 96/00499 in the prior art discloses organoleptically acceptable formulations containing S(+) 1,8-1, 3, 4, 9-tetrahydropyrano [3, 4] indote-1-acetic acid, preferably with an acidic component, also known as S(+)etodolic acid or S(+)etodolac.

The document TR 2007 06886 T4 in the prior art provides a method of treating or preventing a disorder typified by an immunodeficiency (e.g. HIV), wherein the patient is administered a COX-2 inhibitor or derivative or pharmaceutically acceptable salt thereof, preferably diisopropyl fluorophosphate. L-745337, rofecoxib, NS 398, SC 58125, etodolac, meloxicam, celecoxib or nimesulide.

It provides the compositions and products contained in the said invention and consisting of the above compositions or the use of these in preparing the drugs and in the treatment.

The document TR 2001 00107 B in the prior art concerns a multiparticulate, pharmaceutical form with delayed and timed release for starting to make the active principle available 4 to 8 hours after said pharmaceutical form has been ingested, then for gradually releasing the totality of the active principle within the following 8 to 20 hours.

The document TR 2001 00931 relates to a pharmaceutical composition comprising a first component comprising a first active agent dose.

In the said document, on contact with water 70 to 95 % of said dose is released within 3 to 4 hours, and a second component comprising a second active agent dose, a water soluble osmosis inducing agent and a water swellable excipient, said second component having a water permeable coating which, in use on contact with water, ruptures after a delay, and releases the active agent.

The international application WO 00/09091 discloses analgesic controlled release dosage forms comprising (a) 40-80% non-steroid anti-inflammatory drug such as etodolac, (b) 1-15% binder hydroxyethyl cellulose or hydroxypropyl cellulose, (d) 0-1% of ethylcellulose stabiliser, (e) 10-30% of lactose filler, (f) 0,01-10% of a lubricant mixture comprising magnesium stearate and talc however no buffer.

Documents WO 01/19349 and US 4,966,768 also lack a buffer and the first one as well the specific matrix agents.

Document US 4,657,757 describes controlled release oral dosage forms comprising (a) an analgesic-effective amount of acetaminophen, (b) 5-8% hydroxypropyl methylcellulose ether matrix, (c) 10-13dibasic calcium phosphate dihydrate buffer, (d) 4-6% by weight of the polymeric stabiliser mixture of methylcellulose, sodium carboxymethylcellulose or other cellulose ethers, (e) 10-13% filler such as lactose, (f) 1-3% lubricants selected from stearic acid, magnesium stearate, calcium stearate, waxes, polyethylene glycol and magnesium lauryl sulfate however no Etodolac and none of the specific matrix agent/s (b).

### Brief Summary of the Invention

The following objectives are intended under this invention in developing the formulation development method in sustained release form that contains Etodolac:
- Developing an SR (Sustained Release) formula for Etodolac 400 mg SR and Etodolac 600 mg SR,
- Providing and effective analgesic drug that can show effect through sustained release for 24 hours as well as developing an SR (Sustained Release) formula for Etodolac 400 mg SR and Etodolac 600 mg SR.
- Decreasing the daily use dosage by extending the effective period for the said analgesic drug.
- Minimizing the damage induced by the aforementioned systems to gastrointestinal system with the developed drug. (Side effects of the NSAIDs to gastrointestinal system create problems. Instead of taking 3x200 mg or 2x300 mg drugs, an effectiveness of 24 hours will be provided by administering etodolac 600 mg or 400 mg once, and the damage given to the gastrointestinal system will be minimized.)

The present invention solves the above problem by providing a sustained release tablet formulation developed with this invention and that contains Etodolac, which consists of;
a. the active amount of Etodolac (2-(1,8-diethyl-4,9-dihydro-3/fpyrano[3,4- b]indole-1-il) acetic acid) between 20 - 70% in proportion to the total weight of the formulation, which is a non-steroidal anti-inflammatory drug,
b. hydroxylpropyl cellulose or hydroxyethyl cellulose or combination thereof as matrix agents with a proportion of 1-30% to the total weight of the formulation,
c. pharmaceutically acceptable buffer agent with a proportion of 0.5-10% to the total weight of the formulation,
d. pharmaceutically acceptable stabilizing agent with a proportion of 0.5-5% to the total weight of the formulation,
e. pharmaceutically acceptable filler with a proportion of 2.5-40% to the total weight of the formulation,
f. pharmaceutically acceptable lubricant with a proportion of 0.5- 10% to the total weight of the formulation.

### Detailed Description of the Invention

### Description of the drawings

**Figure 1****.a** - Immediate Release (IR) Drug Systems Plasma Concentration / Time Graph
**Figure 1****.b** - Sustained Release (SR) Drug Systems Plasma Concentration / Time Graph
**Figure 2** - Etodolac 400 mg Immediate Release (IR) Tablet (Humber L.G., Medicinal Research Reviews 7(I) 1-28, 1987) and Etodolac 600 mg Sustained Release (SR) Film Tablet Plasma Concentration developed with this invention - Time Graph

In the current invention, Etodolac 400 mg SR film tablet and 600 mg SR film tablet are manufactured with two different applications. One application of the invention is Wet Granulation, and the other application is dry Granulation method.

Wet Granulation Method consists of the following manufacturing steps:
1. Preparing the Granulation Solution
2. Powder Mixture and Wet Granulation
3. Drying and Sieving
4. Adding the lubricant
5. Tablet Press
6. Film Coating
7. Blistering

If Etodolac 400 mg SR or 600 mg SR film tablets that were developed with this invention are to be manufactured with Wet Granulation, granulation solution should be prepared first. After this process, active ingredient Etodolac is granulated by using the filler, binder - matrix agent, buffer agent, stabilizing agent and granulation liquid.

Then the granules obtained are dried and sieved, and then lubricant is added.

After the addition of the lubricant, the mixture obtained is pressed into tablets and coated with film. After film coating, blistering is done and finished product Etodolac 400 mg SR or Etodolac 600 mg SR drugs are obtained.

Dry Granulation consists of the following manufacturing steps:
1. Mixing the active substance and the excipients
2. Passing the powder mixture through the compactor
3. Sieving of the product from the compactor to the desired size
4. Addition of the lubricant to the sieved mixture.
5. Tablet press
6. Film coating
7. Blistering

If Etodolac 400 mg SR or 600 mg SR film tablets that were developed with this invention are to be manufactured with Dry Granulation method, active ingredient Etodolac is mixed first with the excipients without using the aqueous granulation liquid.

The said excipients are the filler materials used in the Wet Granulation method mentioned above, binder - matrix agents, buffer agents and stabilizing agents.

The mixture obtained is in powder form. The said powder mixture is passed through the compactor and the product obtained is sieved to the desired size.

Then lubricant is added to the powder mixture that was obtained after the sieving process and which has smaller particles.

The said lubricants are those used in the Wet Granulation method.

After the addition of the lubricant the said mixture is pressed into tablets and coated with film. Finally, after blistering process, the finished product Etodolac 400 mg SR or 600 mg SR film tablets developed under this invention are obtained.

During the formulation development studies, critical dissolution profile testing was primarily performed. Dissolution profile testing was carried out at pH 1,2 (0,1 N HCl), pH 4,5 Acetate, pH 6,8 Phosphate and pH 7,4 Phosphate media.

As a result of the literature research for the dissolution method, pH 7,4 Phosphate buffer was found to be the most appropriate and selective medium, and was observed to give more appropriate results in comparison with the other media In terms of standard deviation and variation coefficients, and provided a dissolution rate of almost 100%.

In preparing Etodolac 400 mg SR or 600 mg SR film tablet formulations developed with this invention, the hardness of the tablets obtained is 100 - 350 N. However, the hardness of the tablets in question is preferably between 150 - 250 N.

Furthermore, in Etodolac 400 mg SR or 600 mg SR film tablet formulation developed with this invention, after the final product Etodolac 400 mg SR or 600 mg SR film tablets has been applied to a mammal, active plasma concentration belonging to the active ingredient In the body is effective for 1-24 hours. Therefore the said tablets can show effectiveness for 24 hours without the obligation for taking a new tablet during the whole day, provided that the tablets are orally taken once daily.

The tablets produced with Etodolac 400 mg SR or 600 mg SR film tablet formulation developed with this invention is indicated for the treatment of osteoarthritis, rheumatoid arthritis, ankylosing spondylitis symptoms and findings, and also in the treatment of acute gouty arthritis, acute muscular skeletal system pain, post-operative pain and menstrual pain (dysmenorrhea).

The following group of excipients is used as fillers in the granulation process. These excipients are: Microcrystalline cellulose, Polymetacrylates, Cellulose acetate, Calcium carbonate, Calcium phosphate, Calcium sulphate, Lactose monohydrate, Lactose anhydrate, Mannitole, Magnesium carbonate, Magnesium oxide, Starch, Starch pregelatlnlsed.

Binder-matrix agents used for the granulation solution in Wet Granulation method are chosen from Hydroxyethylcellulose, Hydroxypropylcellulose, and combination thereof.

Only one of the said binder - matrix agents or preferably multiple binder - matrix agents can be used in the formulation.

The aforementioned hydroxypropylcellulose has a viscosity of 1500-3000 centipoises (HPC-H) for 1% aqueous solution at 25°C and 75-150 centipoises (HPC-L) for 5% aqueous solution at 25°C.

Hydroxypropylcellulose that have a viscosity of 1500-3000 centipoises (HPC-H) for 1% aqueous solution at 25°C and 75-150 centipoises (HPC-L) for 5% aqueous solution at 25°C, and hydroxyethylcellulose separately constitutes the 5-20% of the total weight of the formulation.

In the said granulation solution, the buffer agent can be chosen from the group that consists of Calcium carbonate, Dibasic sodium phosphate, monobasic sodium phosphate, sodium bicarbonate, sodium citrate dehydrate, sodium hydroxide, tribasic calcium phosphate; and the stabilizing agent can be chosen from the group that consists of Alginic add, Bentonite, Carboxymethyl cellulose calcium, Carboxymethyl cellulose sodium, Glycerin monostearate, Hydroxypropylcellulose, Hypromellose, Aluminum magnesium silicate, Polacrilin potassium, Poloksamer, Polyvinyl alcohol, Potassium chloride, Povidone, Propylene glycol alginate, Sodium alginate, Sodium stearile fumarate.

The excipients added as lubricants to the mixture that were subjected to drying and sieving are chosen from the group that consists of Glycerin behenate, Glycerin monostearate, Calcium stearate, Magnesium stearate, Polyethylene glycol, Poloksamer, Polyvinyl alcohol, Potassium benzoate, Sodium benzoate, Sodium lauryl sulphate, Sodium stearyl fumarate, Stearic acid, Talk.

Weight ratio of Etodolac active substance to the total tablet formulation, used in both of the granulation methods for preparing the Etodolac 400 mg SR or 600 mg SR film tablet formulations developed with this invention is between 1-30%, which is preferably between 5-25%.

Weight ratio of binder - matrix agent to the active substance Etodolac, used in both of the granulation methods for preparing the Etodolac 400 mg SR or 600 mg SR film tablet formulations developed with this invention is between 1-50%, which is preferably between 5-45%.

Weight ratio of buffer agent to the total tablet formulation, used in both of the granulation methods for preparing the Etodolac 400 mg SR or 600 mg SR film tablet formulations developed with this invention is approximately between 0.5-10%, which is preferably between 1-5%.

Weight ratio of stabilizing agent to the total tablet formulation, used in both of the granulation methods for preparing the Etodolac 400 mg SR or 600 mg SR film tablet formulations developed with this invention is between 0.5-5%, which is preferably between 0.5-2.5%.

Weight ratio of the filler agent to the total tablet formulation, used in both of the granulation methods for preparing the Etodolac 400 mg SR or 600 mg SR film tablet formulations developed with this invention is between 2.5-40%, which is preferably between 10-30%.

Weight ratio of the lubricant to the total tablet formulation, used in both of the granulation methods for preparing the Etodolac 400 mg SR or 600 mg SR film tablet formulations developed with this invention is between 0.5-10%, which is preferably between 0.5-5%.

Log linear comparison of Etodolac 400 mg immediate release (IR) tablets in prior art and Etodolac 600 mg sustained release film tablets developed in the current invention is given in Fgure - 2.

As seen from the graph in the Figure - 2, Cₘₐₓ value of Etodolac 400 mg immediate release (IR) tablets in the prior art exceeds the Cₘₐₓ value of Etodolac 600 mg sustained release (SR) tablets developed in the current invention.

However, as seen in the aforementioned graph, blood concentrations of Etodolac 600 mg sustained release (SR) film tablets developed in the current invention keeps above a certain level in blood up to 48 hours, but the blood concentrations of Etodolac 400 mg immediate release (IR) tablets in the prior art reach the peak level in two hours and then quickly decrease.

Since the time needed for reaching the Cₘₐₓ value of Etodolac 600 mg sustained release (SR) film tablets developed in the current invention (tₘₐₓ) is longer, effective time of the product is also longer.

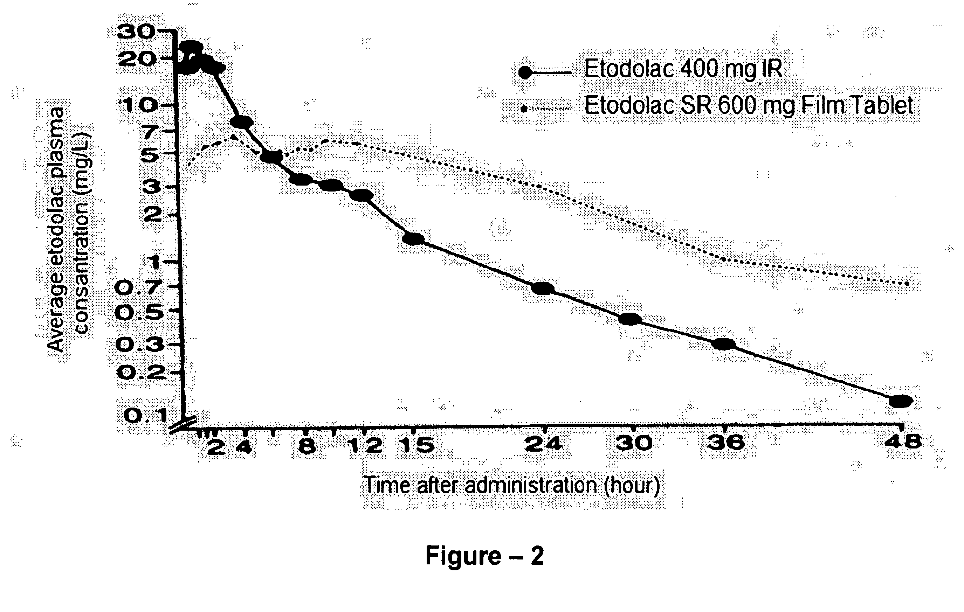

Data relating to the graph of Etodolac 600 mg sustained release film tablets given in Figure 2 are given in Table 2.

**Table 2: Plasma concentration of Etodolac 600 mg sustained release film tablets in the sample taken from the blood.**

| Sampling time from the blood (hours) | Average Plasma concentration (mg/l) |
|---|---|
| 0.0 | 0* |
| 1.0 | 4.5 |
| 2.0 | 5.8 |
| 3.0 | 6.2 |
| 4.0 | 6.7 |
| 5.0 | 5.8 |
| 5.5 | 5.4 |
| 6.0 | 5.4 |
| 6.5 | 5.1 |
| 7.0 | 5.5 |
| 7.5 | 5.3 |
| 8.0 | 5.6 |
| 9.0 | 5.7 |
| 10.0 | 6.4 |
| 12.0 | 6.2 |
| 24.0 | 3.2 |
| 36.0 | 1.0 |
| 48.0 | 0.7 |

| | |
|---|---|
| * = Values under lower limit of quantitation (LLQ) are taken as 0 mg/ml in calculating the average values. | |

In the bioequivalence study carried out on 36 subjects, different Cₘₐₓ (maximum concentration) values are observed at different times (tₘₐₓ) due to the intra-individual differences, and the average Cₘₐₓ value is 8.6 mg/l and tₘₐₓ value is 7.3 hours.

It is important to note here that the plasma concentration against time for Etodolac 400 mg SR or 600 mg SR film tablets developed in the current invention behaves in compliance with the Figure - 1b.

## Claims

1. A sustained release tablet formulation developed with this invention and that contains etodolac, which consists of;
a. the active amount of Etodolac (2-(1,8-diethyl-4,9-dihydro-3*H*-pyrano[3,4-b]indole-1-il)acetic acid) between 20 - 70% in proportion to the total weight of the formulation, which is a non-steroidal anti-inflammatory drug,
b. hydroxylpropyl cellulose or hydroxyethyl cellulose or combination thereof as matrix agents with an proportion of 1-30% to the total weight of the formulation,
c. pharmaceutically acceptable buffer agent with a proportion of 0.5-10% to the total weight of the formulation,
d. pharmaceutically acceptable stabilizing agent with a proportion of 0.5-5% to the total weight of the formulation,
e. pharmaceutically acceptable filler with a proportion of 2.5-40% to the total weight of the formulation,
f. pharmaceutically acceptable lubricant with a proportion of 0.5-10% to the total weight of the formulation.

2. A sustained release tablet formulation that contains etodolac of claim 1, which consists of Etodolac (2-(1,8-diethyl-4,9-dihydro-3*H*-pyrano[3,4-b]indole-1-il)acetic acid) **preferably** between 30-60% in proportion to the total weight of the formulation.

3. A sustained release tablet formulation that contains etodolac of claim 1, which consists of one or more pharmaceutically acceptable binder-matrix agent with a proportion of **preferably** 5-25% to the total weight of the formulation.

4. A sustained release tablet formulation that contains etodolac of claim 1, which consists of one or more pharmaceutically acceptable binder-matrix agent with a proportion of **preferably** 5-45% to the total weight of Etodolac used in the formulation.

5. A sustained release tablet formulation that contains etodolac of claim 1, which consists of a buffer agent with a proportion of **preferably** 1-5% to the total weight of the formulation.

6. A sustained release tablet formulation that contains etodolac of claim 1, which consists of a stabilizing agent with a proportion of **preferably** 0.5-2.5% to the total weight of the formulation.

7. A sustained release tablet formulation that contains etodolac of claim 1, which consists of a filler with an approximate proportion of **preferably** 10-30% to the total weight of the formulation.

8. A sustained release tablet formulation that contains etodolac of claim 1, which consists of a lubricant with a proportion of **preferably** 0.5-5% to the total weight of the formulation.

9. A sustained release tablet formulation that contains etodolac of any of the above claims, in which the filler is chosen from a group of Microcrystalline cellulose, Polymetacrylates, Cellulose acetate, Calcium carbonate, Calcium phosphate, Calcium sulphate, Lactose monohydrate, Lactose anhydrate, Mannitole, Magnesium carbonate, Magnesium oxide, Starch, Starch pregelatinised.

10. A sustained release tablet formulation that contains etodolac of any of the above claims, in which the binder-matrix agent is used in the formulation one by one or preferably more than one.

11. A sustained release tablet formulation that contains etodolac of any of the above claims, in which the buffer agent is chosen from a group of Calcium carbonate, Dibasic sodium phosphate, monobasic sodium phosphate, sodium bicarbonate, sodium citrate dihydrate, sodium hydroxide and tribasic calcium phosphate.

12. A sustained release tablet formulation that contains etodolac of any of the above claims, in which the stabilizing agent is chosen from a group of Alginic acid, Bentonite, Carboxymethyl cellulose calcium, Carboxymethyl cellulose sodium, , Glycerin monostearate, Hydroxypropylcellulose, , Hypromellose, Aluminum magnesium silicate, Polacrilin potassium, Poloksamer, Polyvinyl alcohol, Potassium chloride, Povidone, Propylene glycol alginate, Sodium alginate and Sodium stearile fumarate.

13. A sustained release tablet formulation that contains etodolac of any of the above claims, in which the lubricant is chosen from a group of Glycerin behenate, Glycerin monostearate, Calcium stearate, Magnesium stearate, Polyethylene glycol, Poloksamer, Polyvinyl alcohol, Potassium benzoate, Sodium benzoate, Sodium lauryl sulphate, Sodium stearyl fumarate, Stearic acid and Talk.

14. A sustained release tablet formulation that contains etodolac of any of the above claims, in which hydroxypropylcellulose used in the formulation has a viscosity of 1500-3000 centipoises (HPC-H) for 1% aqueous solution at 25°C and 75-150 centipoises (HPC-L) for 5% aqueous solution at 25°C.

15. A sustained release tablet formulation that contains etodolac of any of the above claims, in which Hydroxypropylcellulose that has a viscosity of 1500-3000 centipoises (HPC-H) for 1% aqueous solution at 25°C and 75-150 centipoises (HPC-L) for 5% aqueous solution at 25°C, and hydroxyethylcellulose separately constitutes the 5-20% of the total weight of the formulation.

16. A sustained release tablet formulation that contains etodolac of any of the above claims, in which the dosage form obtained is a film tablet coated with film.

17. A sustained release tablet formulation that contains etodolac of any of the above claims, in which the tablet forms have a hardness of preferably 150 - 250 N.

18. A process for the production of a sustained release tablet formulation that contains etodolac of any of the above claims, which was produced by two different methods consisting of wet granulation or dry granulation.

19. A process for the production of a sustained release tablet formulation that contains etodolac of claim 18, in which the wet granulation process consists of granulation of Etodolac by using the granulation liquid of the powder mixture obtained by mixing all or some of the excipients consisting of a filler, binder-matrix agent, buffer and stabilizing agent; drying of the granules obtained; addition and mixing of the excipients not added; addition of the lubricant; tablet pressing of the mixture obtained and film coating and blistering.

20. A process for the production of a sustained release tablet formulation that contains etodolac of claim 18, in which, the dry granulation process consists of mixing Etodolac with a filler, binder-matrix agent, buffer and stabilizing agent without using a wet granulation liquid; passing of the powder mixture obtained through a compactor; sieving of the powder mixture to the desired size; addition of the lubricant to sift obtained; tablet pressing of the mixture obtained; film coating and blistering.

21. 400 mg SR or 600 mg SR sustained release tablet formulation that contains etodolac of any of the above claims, for use in the acute and long term treatment of rheumatoid arthritis, osteoarthritis, for the treatment of ankylosing spondylitis symptoms and findings, for the treatment of acute gouty arthritis, acute muscular skeletal system pain, post-operative pain and menstrual pain (dysmenorrheal).

## Patentansprüche

1. Eine Retardtablette-Formulierung entwickelt durch diese Erfindung und welche Etodolac enthält, bestehend aus;
a. der wirksamen Menge von Etodolac (2- (1, 8-diethyl-4,9-dihydro-3-*H*-pyrano [3,4-b] indol-1-il) Essigsäure) 20 - 70% im Verhältnis zum Gesamtgewicht der Formulierung, die eine nicht-steroidale entzündungshemmende Arzneimittel ist,
b. Hydroxyipropylcellulose oder Hydroxyethylcellulose oder einer Kombination davon als Matrixmittel mit einem Verhältnis von 1-30% des Gesamtgewichts der Formulierung,
c. pharmazeutisch akzeptablem Puffermittel, mit einem Verhältnis von 0,5-10% des Gesamtgewichts der Formulierung,
d. pharmazeutisch akzeptablem Stabilisierungsmittel mit einem Verhältnis von 0,5-5% des Gesamtgewichts der Formulierung,
e. pharmazeutisch akzeptablem Füllstoff mit einem Verhältnis von 2,5-40% des Gesamtgewichts der Formulierung,
f. pharmazeutisch akzeptablem Gleitmittel mit einem Verhältnis von 0,5- 10% des Gesamtgewichts der Formulierung.

2. Eine Retardtablette-Formulierung, die Etodolac enthält, nach Anspruch 1, die aus Etodolac (2- (1,8-diethyl-4,9-dihydro-3*H*- pyrano [3,4-b] indol-1-il) Essigsäure) besteht, vorzugsweise zwischen 30-60% im Verhältnis zum Gesamtgewicht der Formulierung.

3. Eine Retardtablette-Formulierung, die Etodolac enthält, nach Anspruch 1, die aus einem oder mehreren pharmazeutisch verträglichen Bindemittel-Matrix-Mittel mit einem Anteil von vorzugsweise 5-25% des Gesamtgewichts der Formulierung, besteht.

4. Eine Retardtablette-Formulierung, die Etodolac enthält, nach Anspruch 1, die aus einem oder mehreren pharmazeutisch verträglichen Bindemittel-Matrix-Mittel mit einem Anteil von vorzugsweise 5-45% des Gesamtgewichts der Etodolac, die in der Formulierung verwendet wird, besteht.

5. Eine Retardtablette-Formulierung, die Etodolac enthält, nach Anspruch 1, die aus einer Puffersubstanz mit einem Anteil von vorzugsweise 1-5% des Gesamtgewichts der Formulierung, besteht.

6. Eine Retardtablette-Formulierung, die Etodolac enthält, nach Anspruch 1, die aus einem Stabilisierungsmittel mit einem Anteil von vorzugsweise 0,5-2,5% des Gesamtgewichts der Formulierung, besteht.

7. Eine Retardtablette-Formulierung, die Etodolac enthält, nach Anspruch 1, die aus einem Füllstoff mit einem ungefähren Anteil von vorzugsweise 10-30% des Gesamtgewichts der Formulierung besteht.

8. Eine Retardtablette-Formulierung, die Etodolac enthält, nach Anspruch 1, die aus einem Gleitmittel mit einem Anteil von vorzugsweise 0,5-5% des Gesamtgewichts der Formulierung besteht.

9. Eine Retardtablette-Formulierung, die Etodolac enthält, nach einem der vorhergehenden Ansprüche, in der der Füllstoff aus einer Gruppe von Mikrokristalline-Cellulose, Polymetacrylates, Celluloseacetat, Calciumcarbonat, Calciumphosphat, Calciumsulfat, Lactose-Monohydrat, Lactoseanhydrat, Mannitole, Magnesiumcarbonat, Magnesiumoxid, Stärke, vorgelierte Stärke.gewählt wird.

10. Eine Retardtablette-Formulierung, die Etodolac enthält, nach einem der vorhergehenden Ansprüche, bei der das Bindemittel-Matrixmittel in der Formulierung eine durch eine oder vorzugsweise mehrere verwendet wird.

11. Eine Retardtablette-Formulierung, die Etodolac enthält, nach einem der vorhergehenden Ansprüche, bei dem das Puffermittel aus einer Gruppe von Calciumcarbonat, dibasisches Natriumphosphat, monobasisches Natriumphosphat, Natriumbicarbonat, Natriumcitrat-Dihydrat, Natriumhydroxid und Tricalciumphosphat. gewählt wird.

12. Eine Retardtablette-Formulierung, die Etodolac enthält, nach einem der vorhergehenden Ansprüche, wobei das Stabilisierungsmittel aus einer Gruppe von Alginsäure, Bentonit, Carboxymethylcellulose-Calcium, Carboxymethylcellulose-Natrium, Glycerin-Monostearat, Hydroxypropylcellulose, Hypromellose, Aluminiummagnesiumsilikat, Polacrilinkalium, Poloksamer, Polyvinylalkohol, Kaliumchlorid, Povidon, Propylenglycolalginat, Natriumalginat und Natriumstearile-Fumarat. gewählt wird.

13. Eine Retardtablette-Formulierung, die Etodolac enthält, nach einem der vorhergehenden Ansprüche, bei der das Schmiermittel aus der Gruppe von Glycerin-Behenat, Glycerin-monostearat, Calciumstearat, Magnesiumstearat, Polyethylenglykol, Poloksamer, Polyvinylalkohol, Kaliumbenzoat, Natriumbenzoat, Natriumlaurylsulfat, Natriumstearylfumarat, Stearinsäure und Talk gewählt wird.

14. Eine Retardtablette-Formulierung, die Etodolac enthält, nach einem der vorhergehenden Ansprüche, wobei in der Formulierung verwendete Hydroxypropylcellulose eine Viskosität von 1500-3000 Centipoise (HPC-H) für 1% ige wässrige Lösung bei 25 ° C und 75-150 Centipoise (HPC-L) für 5% ige wässrige Lösung bei 25 ° C hat.

15. Eine Retardtablette-Formulierung, die Etodolac enthält, nach einem der vorhergehenden Ansprüche, wobei die Hydroxypropylcellulose eine Viskosität von 1500-3000 mPas (HPC-H) 1% ige wässrige Lösung bei 25 ° C und 75-150 Centipoise hat (HPC -L) 5% ige wässrige Lösung bei 25 ° C, und Hydroxyethylcellulose separat bildet die 5-20% des Gesamtgewichts der Formulierung.

16. Eine Retardtablette-Formulierung, die Etodolac enthält, nach einem der vorhergehenden Ansprüche, wobei die erhaltene Dosierungsform eine mit Folie beschichtete Filmtablette ist.

17. Eine Retardtablette-Formulierung, die Etodolac enthält, nach einem der vorhergehenden Ansprüche, wobei die Tablettenformen eine Härte von vorzugsweise 150 bis 250 N haben.

18. Ein Verfahren für Herstellung einer Retardtablette-Formulierung, die Etodolac enthält, nach einem der vorhergehenden Ansprüche, die durch zwei verschiedene Verfahren, die aus Nassgranulierung oder Trockengranulierung besteht, hergestellt wird.

19. Ein Verfahren für Herstellung einer Retardtablette-Formulierung, die Etodolac enthält, nach Anspurch 18, wobei das nasse Granulationsverfahren aus Granulierung von Etodolac unter Verwendung der Granulationsflüssigkeit des durch Mischen aller oder eines Teils der Hilfsstoffe bestehend aus einem Füllstoff, Bindemittel - Matrixmittel, Puffer und Stabilisierungsmittel, erhaltene Pulvermischung; Trocknen der erhaltenen Körnchen; Zugabe und Mischung der nicht hinzugefügten Hilfsstoffe; Zugabe des Schmiermittels; Tablettenpress der erhaltenen Mischung und Folienbeschichtung und Blasenbildung besteht.

20. Ein Verfahren für Herstellung einer Retardtablette-Formulierung, die Etodolac enthält, nach Anspurch 18, wobei das Trockengranulierungsverfahren aus Mischen von Etodolac mit einem Füllstoff, Bindemittel-Matrixmittel, Puffer und Stabilisierungsmittel ohne Verwendung einer Nass-Granulierflüssigkeit; Weitergabe der durch einen Verdichter erhaltene Pulvermischung; Sieben der Pulvermischung auf die gewünschte Größe; Zugabe des Schmiermittels; Tablettenpress der erhaltenen Mischung; Filmbeschichtung und Blasenbildung besteht.

21. 400 mg SR oder 600 mg SR Retardtablette Formulierung, die Etodolac enthält, nach einem der vorhergehenden Ansprüche, für die Benutzung in der akuten und langfristigen Behandlung von rheumatoider Arthritis, Osteoarthritis, für die Behandlung von ankylosierender Spondylitis Symptome und Befunde, zur Behandlung von akuter Gicht Arthritis, akuten Muskelskelettsystem-Schmerzen, postoperativen Schmerzen und Regelschmerzen (Dysmenorrheal).

## Revendications

1. Une formulation à libération prolongée de comprimés mis au point par la présente invention et qui contient l'étodolac, qui consiste en;
a. la quantité efficace d'étodolac (2- (1, 8-diéthyl-4,9-dihydro-3-*H*-pyrano [3,4-b] indole-1-il) d'acide acétique) entre 20 à 70% en proportion du poids total de la formulation, qui est un médicament anti-inflammatoire non stéroïdien,
b. hydroxyipropyl cellulose ou hydroxyéthyle cellulose ou une combinaison de ceux-ci comme un agent de matrice avec une proportion de 1 à 30% du poids total de la formulation,
c. agent tampon pharmaceutiquement acceptable avec une proportion de 0,5 à 10% du poids total de la formulation,
d. agent de stabilisation pharmaceutiquement acceptable avec une proportion de 0,5 à 5% du poids total de la formulation,
e. charge pharmaceutiquement acceptable avec une proportion de 2,5 à 40% du poids total de la formulation,
f. lubrifiant pharmaceutiquement acceptable avec une proportion de 0,5 à 10% du poids total de la formulation.

2. Une formulation à libération prolongée de comprimé qui contient l'étodolac selon la revendication 1, qui consiste en etodolac (2- (1, 8-diéthyl-4,9-dihydro-3*H*-pyrano [3,4-b] indole-1 -il) de l'acide acétique), de préférence entre 30 à 60% en proportion du poids total de la formulation.

3. Une formulation à libération prolongée de comprimé qui contient l'étodolac selon la revendication 1, qui consiste en un ou plusieurs agents pharmaceutiquement acceptable liant-matrice avec une proportion de préférence de 5 à 25% du poids total de la formulation.

4. Une formulation à libération prolongée de comprimé qui contient l'étodolac selon la revendication 1, qui consiste en un ou plusieurs agents pharmaceutiquement acceptable liant-matrice avec une proportion de préférence de 5 à 45% du poids total de Etodolac utilisé dans la formulation.

5. Une formulation à libération prolongée de comprimé qui contient l'étodolac selon la revendication 1, qui consiste en un agent tampon, avec une proportion de préférence de 1 à 5% du poids total de la formulation.

6. Une formulation à libération prolongée de comprimé qui contient l'étodolac selon la revendication 1, qui consiste en un agent de stabilisation avec une proportion de préférence de 0,5 à 2,5% du poids total de la formulation.

7. Une formulation à libération prolongée de comprimé qui contient l'étodolac selon la revendication 1, qui se compose d'une charge avec une proportion approximative de préférence de 10 à 30% du poids total de la formulation.

8. Une formulation à libération prolongée de comprimé qui contient l'étodolac selon la revendication 1, qui consiste en un lubrifiant avec une proportion de préférence de 0,5 à 5% du poids total de la formulation.

9. Une formulation à libération prolongée de comprimé qui contient l'étodolac selon l'une quelconque des revendications précédentes, dans laquelle la charge est choisie dans le groupe de la cellulose microcristalline, polyméthacrylates, l'acétate de cellulose, le carbonate de calcium, le phosphate de calcium, le sulfate de calcium, le lactose monohydraté, anhydrate lactose, Mannitole, le carbonate de magnésium, l'oxyde de magnésium, l'amidon, l'amidon prégélatinisé.

10. Une formulation à libération prolongée de comprimé qui contient l'étodolac l'une quelconque des revendications précédentes, dans lequel l'agent liant-matrice est utilisée dans la formulation, un par un, ou de préférence plus d'un.

11. Une formulation à libération prolongée de comprimé qui contient l'étodolac l'une quelconque des revendications précédentes, dans lequel l'agent tampon est choisi dans le groupe du carbonate de calcium, phosphate de sodium dibasique, le phosphate de sodium monobasique, le bicarbonate de sodium, citrate de sodium dihydraté, l'hydroxyde de sodium et le phosphate de calcium tribasique.

12. Une formulation à libération prolongée de comprimé qui contient l'étodolac l'une quelconque des revendications précédentes, dans lequel l'agent stabilisant est choisi dans le groupe de l'acide alginique, la bentonite, la carboxyméthyl cellulose de calcium, la cellulose carboxyméthyle de sodium, le monostéarate de glycérine, l'hydroxypropylcellulose, l'hypromellose, le silicate de magnésium et d'aluminium, la polacriline potassium, Poloksamer, l'alcool polyvinylique, le chlorure de potassium, povidone, l'alginate de propylène glycol, l'alginate de sodium et le fumarate de sodium stearile.

13. Une formulation à libération prolongée de comprimé qui contient l'étodolac l'une quelconque des revendications précédentes, dans lequel le lubrifiant est choisi dans le groupe de glycérine béhénate, le monostéarate de glycérine, le stéarate de calcium, le stéarate de magnésium, polyéthylèneglycol, Poloksamer, l'alcool polyvinylique, benzoate de potassium, benzoate de sodium, le lauryl sulfate, sodium de la fumarate, l'acide stéarique et talc.

14. Une formulation à libération prolongée de comprimé qui contient l'étodolac l'une quelconque des revendications précédentes, dans laquelle l'hydroxypropylcellulose utilisé dans la formulation a une viscosité de 1500 à 3000 centipoises (HPC-H) pour une solution aqueuse à 1% à 25 ° C et 75-150 centipoises (HPC-L) pour une solution aqueuse à 5% à 25 °C.

15. Une formulation à libération prolongée de comprimé qui contient l'étodolac l'une quelconque des revendications précédentes, dans lequel l'hydroxypropylcellulose qui a une viscosité de 1500 à 3000 centipoises (HPC-H) pour une solution aqueuse à 1% à 25 °C et de 75 à 150 centipoises (HPC -L) pour une solution aqueuse à 5% à 25 °C, et l'hydroxyéthylcellulose constitue séparément le 5-20% du poids total de la formulation.

16. Une formulation à libération prolongée de comprimé qui contient l'étodolac l'une quelconque des revendications précédentes, dans laquelle la forme posologique obtenu est un comprimé de film revêtu d'un film.

17. Une formulation à libération prolongée de comprimé qui contient l'étodolac l'une quelconque des revendications précédentes, dans lequel les formes de comprimés ont une dureté de préférence de 150 à 250 N.

18. Un procédé pour la production d'une formulation à libération prolongée de comprimé qui contient l'étodolac l'une quelconque des revendications précédentes, qui a été produit par deux méthodes différentes consistant en la granulation humide ou granulation à sec.

19. Un procédé pour la production d'une formulation à libération prolongée de comprimé qui contient l'étodolac selon la revendication 18, dans lequel le procédé de granulation humide consiste en une granulation de etodolac en utilisant le liquide de granulation du mélange de poudre obtenu par mélange de tout ou partie des excipients consistant en une charge, agent liant-matrice, agent tampon et agent stabilisant; séchage des granulés obtenus; addition et le mélange des excipients ne est pas joint; addition du lubrifiant; tablette appuyant sur le mélange obtenu ; revêtement de film et faire cloquer.

20. Un procédé pour la production d'une formulation à libération prolongée de comprimé qui contient l'étodolac selon la revendication 18, dans lequel, le procédé de granulation sèche consiste à mélanger etodolac avec une charge, un agent liant-matrice, un agent tampon et un agent stabilisant sans l'aide d'un liquide de granulation par voie humide; passage du mélange de poudre obtenue à travers un compacteur; tamisage du mélange de poudre à la taille désirée; addition du lubrifiant; comprimé pressage du mélange obtenu; revêtement de film et faire cloquer.

21. 400 mg SR ou 600 mg SR formulation de comprimé à libération prolongée contenant l'étodolac l'une quelconque des revendications précédentes, pour utilisation dans le traitement aigu et à long terme de la polyarthrite rhumatoïde, l'arthrose, pour le traitement de la spondylarthrite symptômes de spondylarthrite et les résultats, pour le traitement de goutteuse aiguë arthrite, douleurs musculaires de système squelettique aiguë, la douleur post-opératoire et les douleurs menstruelles (dysmenorrheal).
